# EUROPEAN PATENT APPLICATION

(11) **EP 4 804 207 A1**
(43) Date of publication of application: **09.09.2026**
(21) Application number: 25305311.0
(22) Date of filing: 07.03.2025
(51) Int. Cl.: G16H 50/20, G16H 50/30, G16H 50/70

(54) **AI-ASSISTED STOCHASTIC HYBRID SYSTEM, DEVICE AND METHOD FOR HEALTH AND SAFETY MONITORING AND CAPTURE OF EARLY WEAK SIGNALS OF A RARE EVENT IN A PATIENT**

(71) Applicant: Hope Valley AI, 17000 La Rochelle Cedex 1 (FR)
(72) Inventor: BERDOUZ, Hakima, 17000 La Rochelle (FR)
(74) Representative: Icosa

(57) **Abstract**

The present invention relates to a device (1) and associated computer-implemented method (100) for obtaining at least one trained machine learning model (41) configured at least to predict a risk of occurrence (42), in a predefined time interval, of at least one rare event for a patient, and to a device (2) and associated computer-implemented method (200) for predicting a risk of occurrence (42), in a predefined time interval, of at least one rare event for a patient using the obtained at least one trained machine learning model (41).

## Description

The present invention relates to the field of patient safety enhancement. In particular, the present invention relates to: a device and associated computer-implemented method for obtaining at least one trained machine learning model configured at least to predict a risk of occurrence, in a predefined time interval, of at least one rare event for a patient; and a device and associated computer-implemented method for predicting a risk of occurrence, in a predefined time interval, of at least one rare event for a patient using said at least one trained machine learning model.

Advantageously, the invention uses a hybrid stochastic approach to capture weak signals associated with rare events, thereby enhancing the ability to predict even subtle and low-frequency occurrences. This approach allows to improve patient safety by better detecting rare events through the identification of the weak signals in clinical data.

### BACKGROUND OF INVENTION

Current healthcare systems, despite numerous safety initiatives, still face significant limitations in effectively addressing patient safety concerns. While substantial efforts have been made to minimize the risk of patient harm through investments by government bodies, private organizations, and healthcare institutions, studies continue to report unfavorable patient safety outcomes. Hybrid stochastic systems (HSS), which model the interaction between continuous dynamics, discrete dynamics, and probabilistic uncertainty, have proven valuable for capturing the complexities of such systems. These systems, often applied across various fields such as transportation networks and biological systems can model dynamic interactions with high accuracy. However, in the context of healthcare, existing systems often struggle to reliably predict the risk of occurrence in a predefined time interval of a rare event for a patient with sufficient precision. The primary limitation lies in the challenge of quantifying and assessing the risk of occurrence of a rare event as well as of other events preceding and leading to the rare event (e.g. initiator events, intermediate events, precursor events), which remains a crucial hurdle for improving patient safety. Indeed, this challenge is exacerbated by limited data availability, as rare events occur infrequently, resulting in small sample sizes that reduce the reliability of statistical analysis and predictive modeling. Furthermore, the occurrence of rare events is often influenced by complex external factors such as genetic predisposition, environmental exposures, and interactions between treatments and other unknown risk markers, which may not be fully understood or accounted for in models. Additionally, in machine learning, class imbalance between rare and common events leads to biased models that prioritize the latter, reducing the sensitivity to rare events and further complicating accurate risk quantification. These combined factors contribute to high uncertainty and make it difficult to accurately assess and predict the risk of rare events, impacting efforts to improve patient safety.

Therefore, there is a need for a tool that can reliably estimate and predict the risk of occurrence of a rare event in healthcare, and early characterize its associated weak signals providing actionable insights to minimize harm and enhance patient care outcomes.

The invention falls within this context.

### SUMMARY

This invention thus relates to a device for obtaining at least one trained machine learning model configured at least to predict a risk of occurrence, in a predefined time interval, of at least one rare event for a patient, said device comprising:
- at least one input configured to receive an augmented dataset comprising:
   ∘ a subjects dataset comprising subject data from a plurality of subjects at risk of said at least one rare event and subject data from a plurality of subjects not at risk of said at least one rare event;
   ∘ for each subject of the subjects dataset, a set of parameters associated to said at least one rare event, said rare event being associated to at least one initiator event, and/or at least one intermediate event and/or at least one precursor event, said set of parameters comprising: at least one initiator event parameter and/or at least one intermediate event parameter and/or at least one precursor event parameter;
- at least one processor configured to:
   ∘ cluster said augmented dataset based on at least one parameter of said set of parameters, so as to obtain at least two event clusters, each event cluster of said at least two event clusters being associated to at least one initiator event and/or at least one intermediate event and/or at least one precursor event;
   ∘ compute at least one trajectory of weak signal diffusion between said at least two event clusters, and determine, for each computed trajectory, an associated risk score;
   ∘ compute at least one distance between said rare event and at least one data of each event cluster of said at least two event clusters;
   ∘ train at least one machine learning model using said augmented dataset, said at least one trajectory and the associated risk score, said at least two event clusters and said computed at least one distance, so as to obtain said at least one trained machine learning model, said trained machine learning model being configured to receive at least a patient dataset and a patient set of parameters associated to said rare event and to provide said risk of occurrence, in a predefined time interval, of at least one rare event for said patient ;
- at least one output configured to provide said at least one trained machine learning model.

According to the present invention, an "event" refers to. An event may be defined either in statistical terms as an occurrence or outcome that can be characterized by a risk of occurrence (e.g., probability of occurrence) within a defined system or context or as the happening of a feared threat whose impact would significantly affect the patient's health, safety, and well-being. An event could, for example, be cancer, a recurrence, an adverse therapeutic event, a metastatic form of cancer, a pathological or surgical complication, an allergic shock, therapeutic resistance, a stroke, or death, among others.

In a medical setting, an event refers to a measurable quantity related to a patient's condition, diagnosis, treatment, or physiological response. An event may for example be characterized by its probability of occurrence, its incidence, its frequency, its incubation kinetics, its propagation speed, its capacity for invisibility (e.g., stage 0 cancer that evades the immune system), its multifactorial and/or multidimensional and/or multi-scale nature (e.g., being associated, correlated, dependent, or concurrent with other minor or major events in space and/or time), and the intensity of its impact on the health, safety, and well-being of the patient. This may for instance include disease onset, adverse drug reactions, surgical complications, biomarker detection, or clinical trial outcomes.

A "rare event" refers to an event having a probability of occurrence below a predefined statistical threshold, typically associated with a probability lower than 5%, or even lower than 1% or 0.1%. Beside the probability of occurrence, the rarity of the event is also defined by the severity of the impact associated with the rare event. The combination of a low probability and significant impact distinguishes a "rare event" (rare and severe event) from an "extreme event" (a very rare and very severe event). Thus, a rare event is defined by both its low probability of occurrence and the potential severity of its consequences, while extreme events are characterized by their very low probability and very high severity. In a medical setting, a rare event may for instance correspond to a rare disease is one affecting fewer than 1 in 2,000 individuals (0.05%), a rare adverse drug reaction occurs in fewer than 1 in 10,000 cases (0.01%), and a rare diagnostic complication may have an incidence between 1 in 20,000 and 1 in 100,000 cases (0.001%-0.0001%).

According to one embodiment, a rare event refers to an event having a risk of occurrence below 5%.

Advantageously, the obtained trained machine learning model, through its hybrid stochastic approach, allows for a reliable prediction of the risk of occurrence of a rare event before it happens. By accurately forecasting the likelihood of a rare event, the model allows healthcare providers to intervene proactively. This early intervention is crucial, as it not only helps prevent the escalation of health issues but also reduces the risk of complications, leading to improved patient health outcomes. Additionally, it enhances the precision of medical decisions, ensuring that the right treatment is administered at the right time, thereby optimizing the overall care process and potentially saving the patient's life. In essence, this approach transforms healthcare delivery by enabling timely, data-driven interventions that are tailored to the individual needs of each patient.

Furthermore, the integrated risk-informed combination of the clustering process and the implementation of hybrid stochastic modeling in the training of the machine learning model(s) is notably more efficient and effective compared to existing approaches.

Another advantage of this solution is that it takes into consideration the correlations of the rare event with other events, notably intermediate, precursor, and initiator events, as well as uncertainties related to each type of event, which allows for an accurate prediction of the risk of occurrence of the rare event. In other words, this invention allows also to stochastically model, capture at an early stage and characterize the weak signals of intermediate and initiator events that are precursors to the incubation of a rare and/or extreme event with a very low probability of occurrence.

According to other advantageous aspects of the invention, the device for obtaining at least one trained machine learning model comprises one or more of the features described in the following embodiments, taken alone or in any possible combination.

According to an embodiment, each of said at least one initiator event parameter and/or said at least one intermediate event parameter and/or said at least one precursor event parameter is at least one of: a frequency, a duration, a magnitude, and a time information related to : an initiator event associated with said one initiator event parameter, or an intermediate event associated with said one intermediate event parameter, or a precursor event associated with said one precursor event parameter.

According to an embodiment, said subject data of the subjects dataset comprises for each subject at least one of an age, a genetic risk related to said at least one rare event, a treatment information, and a baseline health score.

According to an embodiment, clustering said augmented dataset comprises using a k-means approach and/or a DBSCAN approach and/or decision trees and/or a random forest.

According to an embodiment, computing at least one trajectory of weak signal diffusion comprises using at least one of: a random walk model (e.g. Markov Chain), a graph-based diffusion model, and a differential equation-based model.

According to an embodiment, computing at least one distance comprises calculating an average distance or a median distance or a midrange distance of said rare event to each data of each event cluster of said at least two event clusters.

According to an embodiment, said at least one machine learning model is at least one of: a Random Forest Classifier, a Gradient Boosting Classifier, a Logistic Regression model, and a SVM classifier.

According to an embodiment, said at least one machine learning model is a survival model and said risk of occurrence, in a predefined time interval, of at least one rare event for a patient is associated with a survival time.

According to an embodiment, said augmented dataset is previously obtained by stochastic simulation.

According to an embodiment, said stochastic simulation comprises:
- adding at least one subject stochastic component to at least one subject data comprised in said subjects dataset; and/or
- adding at least one event stochastic component to at least one of said set of parameters for at least one subject of the subjects dataset.

According to an embodiment, adding at least one subject stochastic component comprises using a random number generator or a Monte Carlo simulation.

According to an embodiment, adding an event stochastic component to said at least one of said sets of parameters comprises using a random event generator.

According to an embodiment, said at least one rare event is breast cancer; said one initiator event parameter is at least one of the following: a personal history of ovarian cancer, a genetic predisposition; said one intermediate event parameter is at least one of the following: a genetic mutation, an exposure to an environmental toxin, a hormonal imbalance; said one precursor event parameter is at least one of the following: an inflammation, an epigenetic change, an uncontrolled cell proliferation.

This invention further relates to a computer-implemented method for obtaining at least one trained machine learning model configured at least to predict a risk of occurrence, in a predefined time interval, of at least one rare event for a patient, said method comprising:
- receiving an augmented dataset comprising:
   ∘ a subjects dataset comprising subject data from a plurality of subjects at risk of said at least one rare event and subject data from a plurality of subjects not at risk of said at least one rare event;
   ∘ for each subject of the subjects dataset, a set of parameters associated to said at least one rare event, said rare event being associated to at least one initiator event, and/or at least one intermediate event and/or at least one precursor event, said set of parameters comprising: at least one initiator event parameter and/or at least one intermediate event parameter and/or at least one precursor event parameter;
- clustering said augmented dataset based on at least one parameter of said set of parameters, so as to obtain at least two event clusters, each event cluster of said at least two event clusters being associated to at least one initiator event and/or at least one intermediate event and/or at least one precursor event;
- computing at least one trajectory of weak signal diffusion between said at least two event clusters, and determine, for each computed trajectory, an associated risk score;
- computing at least one distance between said rare event and at least one data of each event cluster of said at least two event clusters;
- training at least one machine learning model using said augmented dataset, said at least one trajectory and the associated risk score, said at least two event clusters and said computed at least one distance, so as to obtain said at least one trained machine learning model, said trained machine learning model being configured to receive at least a patient dataset and a patient set of parameters associated to said rare event and to provide said risk of occurrence, in a predefined time interval, of at least one rare event for said patient ;
- providing said at least one trained machine learning model.

This invention further relates to a device for predicting a risk of occurrence, in a predefined time interval, of at least one rare event for a patient using at least one trained machine learning model obtained using said device for obtaining at least one trained machine learning model according to any of the herein disclosed embodiments, said device comprising:
- at least one input configured to receive:
   ∘ a patient dataset and a patient set of parameters associated to said rare event;
   ∘ said at least one trained machine learning model ;
- at least one processor configured to predict said risk of occurrence, in a predefined time interval, of at least one rare event for said patient, by feeding said patient dataset and said patient set of parameters associated to said rare event to said at least one trained machine learning model,
- at least one output configured to provide at least said risk of occurrence, in a predefined time interval, of at least one rare event for said patient.

Advantageously, the device for obtaining at least one trained machine learning model and the device for predicting a risk of occurrence, in a predefined time interval, of at least one rare event offer a technology with several advantages:
- a hybrid stochastic approach based on AI, combining stochastic and deterministic models for more precise detection of weak signals associated with rare events;
- real-time monitoring of the patient's health status and early detection of weak signals, facilitating rapid interventions;
- 4D spatiotemporal modeling, integrating 4D visualizations for better understanding of disease progression and rare events;
- a wide range of applications, applicable to a variety of medical conditions, including rare diseases and critical events in oncology.

This invention offers a more comprehensive and integrated approach to patient health monitoring and early detection of rare events, surpassing the limitations of existing solutions. It could have crucial applications in oncology and rare diseases. Here are the potential applications: hybrid AI-stochastic, combining probabilistic and deterministic models to predict incubation weak signals associated with rare events, such as the onset of cancer; monitoring and control, with a real-time automated system to track subtle changes in patients' biomedical data; early detection, enabling detection of weak signals before they become major indicators of a rare event (e.g., malignant tumor); and interactive methodologies and 4D visualization, offering spatiotemporal visualization of the propagation processes of weak signals for specific organs.

According to other advantageous aspects of the invention, the device for obtaining at least one trained machine learning model comprises one or more of the features described in the following embodiments, taken alone or in any possible combination.

According to an embodiment, said risk of occurrence, in a predefined time interval, of at least one rare event for a patient is at least one of: a probability value, a binary value, a percentage, and a Mean Squared Error.

According to an embodiment, said risk of occurrence, in a predefined time interval, of at least one rare event for a patient is compared to a threshold to identify if said patient is at risk of encountering said at least one rare event.

This offers a systematic, objective, and time-specific way to assess a patient's risk of experiencing a rare event. By comparing the computed risk to a threshold, high-risk patients can be identified early, allowing for personalized interventions and improved health outcomes.

According to an embodiment, the risk of occurrence, within a predefined time interval, of at least one rare event for a patient is visualized using a digital model, such as a 4D model. Advantageously, this approach allows visualization of the evolution of the patient's health with time.

This invention further relates to a computer-implemented method for predicting a risk of occurrence, in a predefined time interval, of at least one rare event for a patient using at least one trained machine learning model obtained using said device/computer-implemented method for training at least one machine learning model, said method comprising:
- receiving:
   ∘ a patient dataset and a patient set of parameters associated to said rare event;
   ∘ the at least one trained machine learning model;
- predicting the risk of occurrence, in a predefined time interval, of at least one rare event for the patient, by feeding the patient dataset and the patient set of parameters associated to the rare event to the at least one trained machine learning model,
- providing at least the risk of occurrence, in a predefined time interval, of at least one rare event for the patient.

In addition, the disclosure relates to a computer program comprising software code adapted to perform a method for obtaining at least one trained machine learning model and/or a method for predicting a risk of occurrence, in a predefined time interval, of at least one rare event for a patient, compliant with any of the above execution modes when the program is executed by a processor.

The present disclosure further pertains to a computer-readable medium comprising instructions which, when executed by a computer, cause the computer to carry out the method for obtaining at least one trained machine learning model and/or a method for predicting a risk of occurrence, in a predefined time interval, of at least one rare event for a patient, compliant with any of the above execution modes.

The present disclosure further pertains to a non-transitory program storage device, readable by a computer, tangibly embodying a program of instructions executable by the computer to perform a method for obtaining at least one trained machine learning model and/or a method for predicting a risk of occurrence, in a predefined time interval, of at least one rare event for a patient, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM, an EPROM (Erasable Programmable ROM) or a Flash memory, a portable CD-ROM (Compact-Disc ROM).

### DEFINITIONS

In the present invention, the following terms have the following meanings:

The terms "adapted" and "configured" are used in the present disclosure as broadly encompassing initial configuration, later adaptation or complementation of the present device, or any combination thereof alike, whether effected through material or software means (including firmware).

The term "processor" should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

"Machine learning (ML)" designates in a traditional way computer algorithms improving automatically through experience, on the ground of training data enabling to adjust parameters of computer models through gap reductions between expected outputs extracted from the training data and evaluated outputs computed by the computer models.

A "hyper-parameter" presently means a parameter used to carry out an upstream control of a model construction, such as a remembering-forgetting balance in sample selection or a width of a time window, by contrast with a parameter of a model itself, which depends on specific situations. In ML applications, hyper-parameters are used to control the learning process.

"Datasets" are collections of data used to build an ML mathematical model, so as to make data-driven predictions or decisions. In "supervised learning" (i.e. inferring functions from known input-output examples in the form of labelled training data), three types of ML datasets (also designated as ML sets) are typically dedicated to three respective kinds of tasks: "training", i.e. fitting the parameters, "validation", i.e. tuning ML hyperparameters (which are parameters used to control the learning process), and "testing", i.e. checking independently of a training dataset exploited for building a mathematical model that the latter model provides satisfying results.

A "neural network (NN)" designates a category of ML comprising nodes (called "neurons"), and connections between neurons modeled by "weights". For each neuron, an output is given in function of an input or a set of inputs by an "activation function". Neurons are generally organized into multiple "layers", so that neurons of one layer connect only to neurons of the immediately preceding and immediately following layers.

The above ML definitions are compliant with their usual meaning, and can be completed with numerous associated features and properties, and definitions of related numerical objects, well known to a person skilled in the ML field. Additional terms will be defined, specified or commented wherever useful throughout the following description.

An "event E" may often be considered rare (i.e. rare event) if its probability of occurrence is less than 1% (p(E) < 0.01). This threshold corresponds to a general statistical definition where a phenomenon is unlikely and difficult to model reliably due to the small sample size. In machine learning, a stricter threshold is often used to characterize a rare event in classification (p(E) < 0.05), corresponding to less than 5% of the observations. In some extreme cases, ultra-rare events can be considered with p(E)<0.001, corresponding to less than 0.1% of the observations. This latter threshold is critical for events that are hard to model and require advanced techniques such as oversampling or synthetic data generation.

A rare event can also be defined based on its annual incidence in a given population:
- Rare disease (EU, FDA, WHO): less than 1 case per 2000 (0.05%);
- Rare adverse drug effect: less than 1 case per 10000 (0.01%);
- Rare diagnostic complication: less than 1 case per 20000 - 100000 (0.001% - 0.0001%).

In some fields, extreme value models are used to define rare events. A common approach is to consider that p(E) < 1/N, where N is the total size of the population of interest. For example: If 100000 patients are studied, an event is considered rare if p(E)<10⁻⁵.

In preventive oncology, an early-stage cancer with a prevalence of 0.01% (1 in 10000) may be considered extremely rare.

In risk engineering applied to medicine, a rare event must be defined by considering:
- Its probability of occurrence (p(E)),
- Its clinical impact, characterized by the severity of its consequences;
- The degree of uncertainty associated with its estimation, characterized by the limited number of observed cases.

In such cases, adjustments can be made based on the clinical context, using Bayesian methods to integrate data uncertainty.

### BRIEF DESCRIPTION OF THE DRAWINGS

The present disclosure will be better understood, and other specific features and advantages will emerge upon reading the following description of particular and non-restrictive illustrative embodiments, the description making reference to the annexed drawings wherein:
**Figure 1** is a block diagram representing schematically a particular mode of a device for obtaining at least one trained machine learning model, compliant with the present disclosure;
**Figure 2** is a flow chart showing successive steps of the computer-implemented method executed by the device of figure 1;
**Figure 3** is a block diagram representing schematically a particular mode of a device for predicting a risk of occurrence, in a predefined time interval, of at least one rare event for a patient using at least one trained machine learning model obtained using the device of figure 1, compliant with the present disclosure;
**Figure 4** is a flow chart showing successive steps of the computer-implemented method executed by the device of figure 3;
**Figure 5** diagrammatically shows an apparatus integrating the functions of the devices of figures 1 and 3.

On the figures, the drawings are not to scale, and identical or similar elements are designated by the same references.

### ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

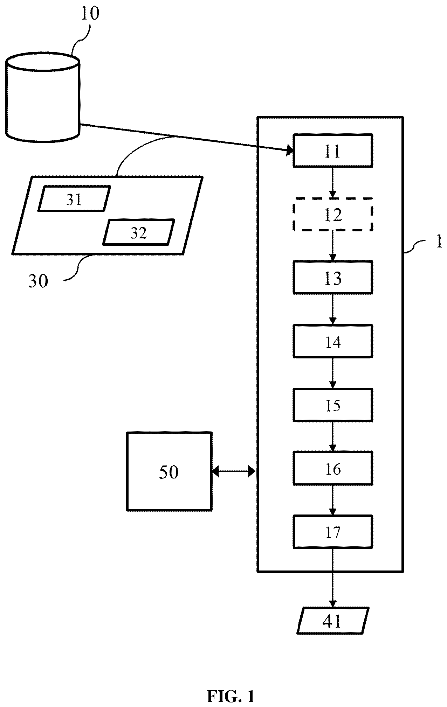

The present disclosure will be described in reference to a particular functional embodiment of a device 1 for obtaining at least one trained machine learning model, as illustrated on **Figure 1****.**

The device 1 is adapted for obtaining at least one trained machine learning model 41 configured at least to predict a risk of occurrence 42 in a predefined time interval of at least one rare event for a patient (i.e. risk of occurrence 42 of the rare event).

The rare event may be, for example, a therapeutic rare event (e.g., adverse drug reaction, adverse reaction to chemotherapy), a pathological rare event (e.g., sudden cardiac arrest, aggressive progression of breast cancer, unexpected metastasis in ovarian cancer), or an interventional rare event (e.g., complications during imaging or surgery, post-operative complications following mastectomy or hysterectomy).

The predefined time interval for predicting the risk of occurrence 42 of the rare event may vary depending on the clinical context and the type of rare event considered. For example, it may be a short-term interval, such as 6 months or 12 months, for events like adverse drug reactions or post-operative complications. Alternatively, it may be a medium- to long-term interval, such as 12 to 60 months, for events like cancer recurrence, cancer progression, or late-onset treatment side effects. In some cases, the interval may extend beyond 60 months, particularly for predicting long-term outcomes such as late metastasis or chronic therapy-related complications.

The device 1 may comprise at least one input configured to receive (cf. module 11) an augmented dataset 30 comprising:
- a subjects dataset 31 comprising subject data collected on subjects identified to be at risk of having said at least one rare event and subject data from subjects identified to be not at risk of having said at least one rare event; and
- for each subject of the subjects dataset 31, a set of parameters 32 (e.g., at least two parameters) associated to said at least one rare event. Notably, the set of parameters 32 may comprise a first subset of parameters comprising at least one initiator event parameter and/or a second subset of parameters comprising at least one intermediate event parameter and/or a third subset of parameters comprising at least one precursor event parameter.

A subject identified at risk of having/experiencing a rare event may be for instance:
- a subject who has already experienced the rare event;
- a subject who already exhibited an initiator event, an intermediate event, or a precursor event;
- a subject classified at risk based on an outcome of an algorithm or health related questionnaire;
- a subject who has been exposed to a known external risk factor, such as environmental hazards, lifestyle factors, or treatment-related risks;
- a subject belonging to a demographic or clinical subgroup with a statistically higher incidence of the rare event.

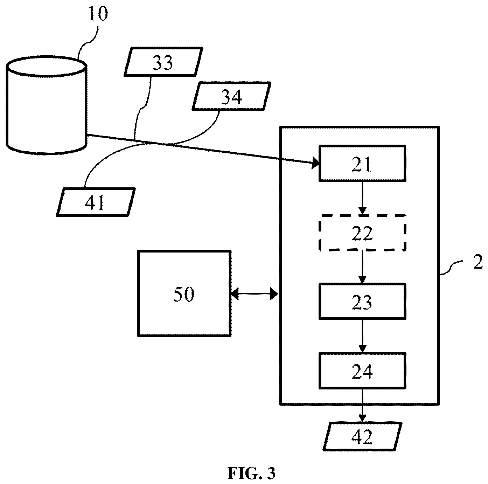

The device 1 for obtaining at least one trained machine learning model 41 is associated with a device 2 (cf. **Figure 3****)** for predicting a risk of occurrence 42, in a predefined time interval, of at least one rare event for a patient using the trained machine learning model 41 obtained with device 1.

Though the presently described devices 1 and 2 are versatile and provided with several functions that can be carried out alternatively or in any cumulative way, other implementations within the scope of the present disclosure include devices having only parts of the present functionalities.

Each of the devices 1 and 2 is advantageously an apparatus, or a physical part of an apparatus, designed, configured and/or adapted for performing the mentioned functions and produce the mentioned effects or results. In alternative implementations, any of the device 1 and the device 2 is embodied as a set of apparatus or physical parts of apparatus, whether grouped in a same machine or in different, possibly remote, machines. The device 1 and/or the device 6 may e.g. have functions distributed over a cloud infrastructure and be available to users as a cloud-based service, or have remote functions accessible through an API.

The device 1 and the device 2 may be integrated in a same apparatus or set of apparatus, and intended to same users. In other implementations, the structure of device 1 may be completely independent of the structure of device 2, and may be provided for other users.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the device 1 or of the device 2.

The device 1 comprises a module 11 for receiving the augmented dataset 30 comprising: a subjects dataset 31 and a set of parameters 32, for each subject of the subjects dataset, the parameters being associated to the rare event. The augmented dataset 30, including the subjects dataset 31 and the set of parameters 32 may be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). Alternatively, the augmented dataset 30, the subjects dataset 31, and the set of parameters 32 may be received from a communication network.

The subjects dataset 31 may include subject data from two groups:
- Subject data from a plurality of subjects at risk of the rare event (i.e., subjects at risk of experiencing/encountering the rare event) may comprise a combination of: e.g., patients who underwent chemotherapy, patients who experienced a type of cancer different from the one associated with the rare event (e.g., patients who had ovarian cancer and for whom the risk of developing breast cancer is being predicted), patients receiving radiation therapy, patients with BRCA1/2-mutated ovarian cancer, patients who experienced/encountered the rare event, etc.;
- Subject data from a plurality of subjects not at risk of the rare event (i.e., subjects unlikely to experience/encounter the rare event) may comprise a combination of: e.g., patients who underwent chemotherapy without developing any toxicity, patients who had ovarian cancer but did not develop subsequent breast cancer, patients receiving radiation therapy without experiencing adverse effects, patients with BRCA1/2 mutations who remained disease-free over a long-term follow-up period, patients who underwent mastectomy for whom the risk of developing breast cancer is eliminated, patients who underwent ovariectomy for whom the risk of developing ovarian cancer is eliminated, etc.

"Subject data" refer to information related to a patient's health, collected from various sources, including routine medical check-ups, electronic health records (EHRs), patient self-reported data, wearable medical devices, laboratory tests, imaging studies, genetic analysis, clinical trials, observational studies, and real-time monitoring systems.

Subject data can come in various formats, including numerical, text, categorical, image, audio, video and time-series data.

For instance, subject data may comprise a baseline health score (e.g. obtained from a questionnaire, from risk markers, from a tool for assessing the risk of developing breast and ovarian cancer such as CanRisk; demographics like identity, age, genetic risk; diagnoses and treatment information (e.g. hormone replacement therapy for menopause); lab test results, such as blood tests measuring tumor markers or genetic testing for mutations; clinical intervention data, like records of chemotherapy or surgical treatments and associated treatment side effects; lifestyle and environmental data, including information about diet, smoking habits, or exposure to environmental toxins.

Advantageously, the variety of types of data of the subjects dataset 31 allows to provide a comprehensive view of the subjects, enabling a reliable prediction of the risk of occurrence 42 of the rare event.

The augmented dataset 30 may comprise, for each subject of the subjects dataset 31, a set of parameters 32 associated with the rare event.

Advantageously, the set of parameters 32 may capture different stages leading to the occurrence of the rare event, since it may comprise at least one of initiator event parameter(s), intermediate event parameter(s) or precursor event parameter(s)

Initiator event parameters are associated to an initiator event. According to the invention, an initiator event is an identifiable occurrence or condition that triggers or increases the likelihood of occurrence of a rare event. It serves as an early trigger (i.e., that occurs before an intermediate event and a precursor event) that sets off a chain of events leading to the rare event, In a medical setting, an initiator event can be a physiological change, an external exposure, a genetic mutation, a medical intervention, or an unexpected patient response that sets off a chain reaction resulting in a rare outcome. For example, in breast cancer, an initiator event could be the detection of a genetic predisposition (e.g., BRCA1 mutation) that increases the patient's risk of aggressive breast cancer progression. The associated initiator event parameter could be a quantifiable value such as the presence or absence of the BRCA1 mutation (e.g. binary value), a genetic risk score (e.g. between 0 and 100) or a measure of the intensity of the mutation (e.g., a score based on the severity of the mutation, such as 0 for low-impact mutations, 1 for moderate-impact mutations, and 2 for high-impact mutations). For a patient with a genetic risk factor like a family history of breast cancer, the associated parameter could include the number of first-degree relatives diagnosed with breast cancer before a certain age or a family risk score based on genetic models. Other possible initiator event parameters may be mammographic density (e.g. quantified as low (0), moderate (0.5), or high density(1)), which is known to increase the risk of breast cancer. Advantageously, an initiator event parameter provides early indicators of potential risk, enabling more accurate prediction of the risk of occurrence 42 of the rare event;

Intermediate event parameters are associated with an intermediate event. According to the invention, an intermediate event may represent a situation that occurs after the initiator event but before the rare event. It acts as a transitional or intermediary stage (i.e., occurring after an initiator event and before a precursor event), which may influence the progression or likelihood of the rare event.. In a medical setting, an intermediate event could represent a physiological change, a response to a treatment, or a clinical manifestation that increases or modifies the risk of occurrence of the rare event. For example, in the case of metastatic breast cancer as the rare event, an intermediate event may be a drop in white blood cell count, signaling a possible increased risk of infection. The associated intermediate event parameter could be a quantifiable value such as the change in white blood cell count (e.g., a percentage decrease from baseline), which may indicate a compromised immune system. Other intermediate event parameters may be: weight loss beyond a certain threshold (e.g., greater than 5% weight loss over a 6-month period); tumor growth rate during chemotherapy, measured as the percentage increase in tumor size between imaging scans (e.g., a 20% increase in tumor size over a 3-month period). Advantageously, intermediate event parameters may help to identify the progression of potential risks before they manifest into a rare event, allowing for timely interventions to prevent the occurrence of the rare event;

Precursor event parameters are associated with a precursor event. According to the invention, a precursor event may represent a late-stage indicator (i.e., occurring after an initiator event and an intermediate event), that signals an imminent occurrence of the rare event. For example, in breast cancer, a precursor event could be a steady increase in tumor markers, such as CA-125 or CEA, which are often used to monitor cancer recurrence. The associated precursor event parameter may be a quantifiable value such as the rate of increase in tumor markers (e.g., a percentage increase in CA-125 levels over a specified period, like a 30% increase in 3 months), or the absolute value of the marker exceeding a predefined threshold (e.g., CA-125 > 35 U/mL). Another example could be radiological changes in imaging studies, such as CT scans or MRIs, where an increase in tumor size or the appearance of new lesions could act as a precursor event. The associated precursor event parameter could be a measurement of tumor size progression (e.g., a tumor growth rate of >10% over 6 months). Other precursor parameters might include persistent or worsening symptoms like pain, fatigue, or changes in physical functioning that are beyond typical expectations, which could be quantified through patient-reported outcome scores, notably from health questionnaires (e.g., a score of 7 or higher on a scale of 1-10). Advantageously, precursor event parameters provide critical signals that the rare event may be imminent, enabling healthcare professionals to intervene immediately to prevent the occurrence of the rare event.

The format of a parameter can vary widely depending on its type and the specific context in which it is applied. It may be represented as a numerical value, such as an integer or floating-point number, to quantify characteristics like temperature, dosage, or concentration. In other cases, a parameter could take the form of text, such as names or descriptions, to represent categorical or textual information. Some parameters may be binary, represented by boolean values like true/false or 0/1, which indicate specific states or conditions. Date or time parameters may be used to track occurrences or measurements, such as recording the time of an event or the start of treatment. Additionally, parameters may involve predefined categories or labels. Parameters may also take the form of complex data, structured in the form of arrays, lists, or even dictionaries to capture multiple values or relationships. In certain contexts, parameters may also be represented graphically or visually, such as in images or graphs, or be expressed with associated units of measurement.

An initiator event parameter, associated to an initiator event, may be a frequency, a duration, a magnitude, or a time information related the initiator event. An intermediate event parameter, associated to an intermediate event, may be a frequency, a duration, a magnitude, or a time information related the intermediate event. A precursor event parameter, associated to a precursor event, may be a frequency, a duration, a magnitude, or a time information related the precursor event.

Frequency may refer to how often an event occurs over a specific time period. For example, if an initiator event is related to a subject's exposure to a certain risk factor, the frequency could represent how many times the subject encounters that risk factor within a defined period (e.g., 3 times per year).

Duration may refer to how long the event lasts when it occurs. For example, if the event is a health episode, the duration would represent the length of time the health episode lasts, such as 2 weeks or 5 hours.

Magnitude may refer to a risk attenuator/amplifier, indicating how much the event either reduces (attenuates) or increases (amplifies) the risk of occurrence 42 of the rare event, either internally (e.g. in direct relationship with the subject) or externally (e.g. due to external/environmental factors). For instance, exposure to environmental toxins such as carcinogens could amplify the risk of ovarian cancer, while a subject's immune response or genetic factors might attenuate the risk of cancer by slowing tumor progression or improving the body's ability to fight off abnormal cell growth. In another example, magnitude may refer to the strength of the event. It may measure how intense or severe the event. If an event is a symptom for instance, the magnitude could indicate the severity level of the symptom (e.g., mild, moderate, or severe); if the event is a chemical exposure, the magnitude could represent the concentration of the chemical.

Time information may refer to the timing of the event, such as when the event occurs in relation to other events or time checkpoints. It could involve precise time data, such as the exact date and time of an event, or relative time information, like "one month before an intermediate event." .

Advantageously, incorporating initiator and/or intermediate and/or precursor event parameters in the augmented dataset allows the machine learning model to identify patterns across various stages of a subject's conditions, and therefore enables more accurate predictions of the risk of occurrence 42 of the rare event for the patient(s) at risk of the rare event, facilitating early interventions and potentially preventing adverse outcomes in high-risk patient(s).

In an example, the initiator, intermediate and precursor events may be obtained from threat scenarios. For example, a threat scenario may involve a sequence of events where the initiator event is a genetic mutation (e.g., BRCA1), the intermediate event is the formation of cysts in the ovaries, and the precursor event could be the development of abnormal cell growth leading to the formation of a tumor, ultimately culminating in ovarian cancer (rare event).

The examples of initiator, intermediate, and precursor event(s), along with their associated initiator/intermediate/precursor event(s) parameters provided above, are intended solely for illustrative purposes and should not be considered exhaustive or restrictive. The key point is that there may be various types of events that may manifest in different ways and at varying time points depending on the rare event. It is important to recognize the chronological order of events, where each stage (initiator/intermediate/precursor) plays a different role in the progression (e.g. the speed of progression) toward the rare event. Therefore, the augmented dataset 30 allows taking into consideration this variability for an adequate timely intervention improving patient safety and outcomes.

In an embodiment, the augmented dataset 30 may be previously obtained by stochastic simulation. In other words, the augmented dataset 30 may be further completed by generating synthetic subject (e.g., synthetic subject data) that mimic real-world medical conditions and responses within the augmented dataset 30 and/or synthetic parameters. Synthetic subject generation may be performed by incorporating a random or probabilistic element (stochastic component) into the data of at least one subject in the subjects dataset 31. Synthetic parameters may be obtained by incorporating a random or probabilistic element (stochastic component) into the set of parameters 32 associated with the subject.

The augmented dataset 30 generation may be performed upstream the training process by device 1 or another device, communicatively coupled to device 1.

In an embodiment, stochastic simulation may comprise:
- adding at least one subject stochastic component to at least one subject data comprised in said subjects dataset 31; and/or
- adding at least one event stochastic component to at least one of said set of parameters 32 for at least one subject of the subjects dataset 31.

To add a subject stochastic component, for example, if the first subject's data is age, the second subject's data is genetic risk, and the third subject's data is baseline health score, random noise can be introduced. For age, a stochastic component (e.g., ±10 years) could be added, simulating age variability, resulting in a value like 30 or 50 for a subject with an initial age of 40. For genetic risk, a Monte Carlo simulation may be used to introduce variability; for instance, if the subject's genetic risk score is 0.8, a random variation within a defined range (e.g., ±0.1) could generate a new score between 0.7 and 0.9, reflecting the uncertainty in genetic risk prediction. For the baseline health score, Gaussian noise may be added to account for health measurement uncertainty, where a score of 75 might become 77.2 or 72.8 by introducing random Gaussian-distributed noise with a mean of 0 and a higher standard deviation, such as 2.0, to capture more substantial natural variation in health assessments. This approach simulates broader fluctuations in the subject's health status, making the data more reflective of real-world variability. These methods allow for more realistic, variable subject data.

Advantageously, adding a subject stochastic component incorporates variability to the augmented dataset and allows to obtain a larger database for training which could improve the performance of the trained machine learning model 41.

To add an event stochastic component, randomness may be introduced into the timing, occurrence, and impact of the event. Random timing refers to the uncertainty in when an event occurs, and this may be modeled using a Poisson distribution for instance, where the time between two consecutive events follows a random rate. For example, if a health deterioration event is simulated, the event time may be determined by this Poisson process, meaning the occurrence of health deterioration is spread over time according to a certain rate, with each subject potentially experiencing it at a different time point. Probabilistic occurrence is about the probability of an event happening based on a subject's risk factor. For instance, a subject with a high-risk factor (e.g., a genetic predisposition or poor baseline health score) would have a higher probability of experiencing the health deterioration event, which can be modeled using a Bernoulli distribution or other probability functions to represent this risk. Finally, random impact refers to the unpredictable change an event can cause to a subject's parameter. For example, the impact of the health deterioration event on a subject's health score may be modeled using a Gaussian distribution, meaning the change in the health score will vary randomly, with a mean of 0 and a standard deviation representing a typical fluctuation. Advantageously, adding an event stochastic component as such allows to take into consideration variability and uncertainty associated with events in real-world scenarios.

Adding an event stochastic component may comprise using a random event generator. A random event generator may introduce stochasticity into at least one parameter of a set of parameters 32, ensuring variability in the augmented dataset 30. Stochasticity may be introduced in event timing (e.g. randomizing when the event occurs using probability distributions like the Poisson process for event frequency), event occurrence (e.g. probabilistically determining whether an event happens, for instance, using the Bernoulli distribution for binary events), event intensity (e.g. varying the impact of an event, such as health deterioration, modeled with a Gaussian distribution for random intensity variations), and event propagation (e.g. introducing randomness in how events spread across subjects). The random event generator may use various probability distributions to model these components, such as the Bernouilli distribution to model if an event happened or not (e.g. binary value, yes/no), the Poisson process to model the number of events occurring within a period of time, Gaussian to generate variations in event intensity, and Exponential distribution to model the waiting time between events. The event generator randomizes event parameters, including the event type (e.g., minor health anomaly vs. major deterioration), time of occurrence (e.g., next month, next year), impact severity (e.g., health score reduction), and the subject affected (e.g. randomly selected based on risk factors). Advantageously, this allows for realistic, dynamic simulations of events that behave unpredictably across subjects, capturing real-world uncertainties.

The device 1 further comprises optionally a module 12 for preprocessing the received augmented dataset 30. The module 12 may notably be adapted to standardize, scale or normalize the subjects dataset 31 and/or the sets of parameters 32 for sake of efficient and reliable processing. Standardization/normalization may be particularly useful when the augmented dataset 30 comprises data originating from different sources (e.g. genetic data, questionnaires data, imaging data). This can be done for instance using techniques such as z-score normalization, min-max scaling, log transformation for skewed data, and feature embedding methods to harmonize diverse data types into a common analytical framework. It may perform additional preprocessing tasks like data imputation, data augmentation, noise reduction, or dimensionality reduction. According to various configurations, the module 12 is adapted to execute only part or all of the above functions, in any possible combination, in any manner suited to the following processing stage. Thanks to standardization, differences between sources may then be neutralized or minimized. This may make the device 1 more efficient and reliable.

The device 1 may further comprise a module 13 for clustering (e.g. through supervised, unsupervised, semi-supervised clustering method) the augmented dataset 30 based on at least one parameter of the set of parameters 32. At least two event clusters are thus obtained, wherein each event cluster comprises subject data grouped according to shared characteristics based on the parameters in the set of parameters 32. Specifically, one cluster may comprise subject data linked to at least one initiator event; another cluster may comprise subject data associated with at least one intermediate event; and a third cluster may comprise subject data corresponding to at least one precursor event. Alternatively, the cluster may comprise subject data linked to at least one initiator event and at least one intermediate event or to at least one initiator event and at least one precursor event, or to at least one intermediate event and at least one precursor event, or to at least one initiator event, at least one intermediate event, and at least one precursor event. Module 13 may cluster the augmented dataset 30 using techniques like k-means clustering, DBSCAN, decision trees, or random forests so as to obtain distinct event clusters.

For instance, in the k-means approach, the augmented dataset 30 may be divided into k clusters (k ≥ 2, k integer) by minimizing the distance between subjects that share similar parameters (e.g., BRCA1 mutation and ovarian cysts in one cluster). A k-means algorithm may work iteratively to minimize the within-cluster sum of squared errors (SSE), which is the sum of squared distances between a subject's feature vector and the centroid of its assigned cluster. Each subject's feature vector may include parameters such as genetic mutations (e.g., BRCA1), intermediate events (e.g., ovarian cysts), and precursor events (e.g., fatigue). Practically, k centroids may be chosen randomly, and each subject may be assigned to the cluster whose centroid is closest in terms of Euclidean distance. The centroids are then recalculated as the mean of all subjects assigned to each cluster. The process repeats until the centroids no longer change significantly, indicating convergence. For example, subjects with BRCA1 mutations and ovarian cysts might form a cluster, while subjects with different genetic mutations and distinct intermediate events (e.g., abnormal cell growth) may form separate clusters. In another example, one cluster could group subjects with genetic mutations (e.g., BRCA1) and intermediate events like ovarian cysts, while another could group subjects with environmental exposures leading to conditions like ovarian tumors.

In another example, a decision trees algorithm may build a model by recursively splitting the augmented dataset 30 based on the values of a parameter (e.g., genetic mutations like BRCA1, family history of ovarian cancer, environmental exposure, etc.). At each node, the algorithm may select the feature (i.e. parameter) that maximizes information gain or minimizes impurity/entropy to split the augmented dataset 30 in the most informative way. For example, at a first decision node, the tree might first check if a subject has the BRCA1 mutation. If the answer is yes, the algorithm may split the data into two branches: one for subjects with the mutation and another for those without it. The process continues recursively for each branch, where the algorithm evaluates other features, like whether the subject has a family history of cancer or has developed ovarian cysts, to split the data further. Each leaf node of the tree corresponds to a prediction of the risk of occurrence 42 of the rare event (e.g., whether the subject is at high risk of developing ovarian cancer), based on the accumulated splits and decision rules. A decision rule may be for example: if BRCA1 mutation is present (e.g. binary value of 1) and a family history of cancer is reported (e.g. binary value of 1), then associate high risk of breast cancer (e.g. binary value of 1). Another decision rule may be: if BRCA1 mutation is absent (e.g. binary value of 0) and abnormal ovarian cell growth is not detected (e.g. binary value of 0), then associate low risk of ovarian cancer (e.g. binary value of 0). In these examples, it is supposed that the decision tree uses binary values (1 for presence, 0 for absence) to classify the subject's risk based on the conditions met. In other examples, probability values, percentage values, or other continuous classifications may be used instead of binary values, allowing for a more nuanced risk assessment.

Advantageously, clustering enables the identification of distinct patterns in the augmented dataset 30 and provides help in understanding relationships between different types of events.

The device 1 may further comprise a module 14 for computing at least one trajectory of weak signal diffusion between the at least two event clusters. In other words, the movement, spread, or influence of weak signals as they propagate between event clusters over time are analyzed and tracked. This involves identifying and modeling how subtle, often unnoticed signals (which could be early indicators of change or emerging trends) diffuse and influence event clusters, and predicting how these signals might evolve or manifest in the future.

Module 14 is then further configured to determine, for each computed trajectory of weak signal diffusion, an associated risk score. Module 14 may use for instance, a random walk model (e.g. Markov Chain), a graph-based diffusion model, or a differential equation-based model.

To compute a trajectory of weak signal diffusion between event clusters and associate a risk score, weak signals are modeled as early risk indicators that spread through a network, either between subjects or within the same subject over time. Diffusion models, such as Epidemiological models (SIR), Graph-based propagation models (PageRank, Influence Spread), and Markov Chains for state transitions, describe how these signals propagate. The risk score quantifies the likelihood that a weak signal transition from Cluster 1 to Cluster 2 will result in a high-risk state (e.g., severe health condition). This score may be derived from: 1) transition probabilities between clusters (using a Markov model), 2) signal strength decay (via a diffusion kernel), and 3) event impact score (severity measure). To calculate weak signal diffusion trajectories, a graph representation is constructed, where nodes represent events, and edges represent connections between events based on time or subject similarity (e.g., using cosine similarity or Euclidean distance). Transition probabilities are then computed (using a Markov model), a trajectory is derived, and a risk score is assigned to each trajectory.

For example, in a graph-based diffusion model, the trajectory between clusters may be computed by representing subjects as nodes and edges as weighted connections based on the strength of the relationship between precursor events (e.g., chronic inflammation, hormonal imbalance) and intermediate events (e.g., abnormal ovarian cell growth). The edge weights may be derived from statistical correlations, such as the probability of subjects with precursor events progressing to intermediate events. Risk propagation may be simulated using probability-based diffusion, where risk spreads through the network based on connectivity patterns. If many subjects with precursor events transition to an intermediate event, the connection strength between these clusters increases, reinforcing the trajectory. This diffusion process enables the system to dynamically adjust risk scores and refining events progression assessment. For instance, in a graph-based diffusion model, at least one trajectory is computed between each pair of clusters. If there are more than two clusters, several trajectories may be calculated for every possible pair of clusters, allowing to analyze how risk spreads across different event clusters.

Determining the risk score associated with a computed trajectory may be achieved using the following equation: R_{score} = Σ_{i,j} p(i, j). S_{diffusion}. I_{impact} , where p(i,j) is the transition probability from event i to event j, S_{diffusion} is the diffusion strength, and I_{impact} is the severity score of the destination event. A risk score (R_{score}) may be determined for multiple trajectories. The trajectory with the higher risk score would indicate a higher likelihood of transitioning to a high-risk or severe health condition. In this manner, comparing multiple trajectories involves calculating the risk score for each possible trajectory and potentially selecting those with higher risk scores as the primary focus for further analysis or intervention. This enables a more detailed understanding of how risk transitions from one event to another within the network.

The device 1 may further comprise a module 15 for computing at least one distance between the rare event and at least one data of each event cluster of the at least two event clusters. This distance computation may be done by measuring for instance the average, median, or midrange distance from the rare event to each data point in an event cluster. For example, module 15 may calculate the average distance between the rare event and all the data points in a precursor and intermediate event clusters, or use the median distance to reduce the impact of outliers. The distances help measure how similar or dissimilar the rare event is to the data patterns or characteristics of the event clusters. If the rare event shares many similar features with a particular cluster's data (i.e., low distance), it suggests a higher likelihood or a closer connection to the patterns or risk factors within that cluster. On the other hand, a high distance indicates that the rare event is significantly different from the data in that cluster, suggesting a lower likelihood of association. This allows for quantifying how far or close the rare event is in relation to the other event clusters, providing a measure of risk proximity based on event data.

The device 1 may further comprise a module 16 for training at least one machine learning model using the augmented dataset 30, the at least one trajectory and the associated risk score, the at least two event clusters and the computed at least one distance, so as to obtain at least one trained machine learning model 41. The obtained trained machine learning model 41 may be configured to receive at least a patient dataset 33 and a patient set of parameters 34 associated to the rare event and to provide the risk of occurrence 42, in a predefined time interval, of at least one rare event for the patient.

The at least one machine learning model may be one of: a Random Forest Classifier, a Gradient Boosting Classifier, a Logistic Regression model, a SVM classifier.

According to an embodiment, a machine learning model may be a survival model such as a Cox model, a Kaplan-Meier Model, or Random Survival Forests. In this case, the risk of occurrence 42 of the rare event may be associated with a survival time, which represents the estimated time until the event occurs relatively to the moment of the prediction of the risk of occurrence 42 of the rare event. In other words, the obtained trained machine learning model 41 may be configured to provide the risk of occurrence 42 of the rare event and an associated survival time. For example, in case of breast cancer, the survival model may predict the probability that a patient will develop metastasis within the next 36 months. Similarly, for a therapeutic rare event, the survival model may estimate the likelihood of a severe adverse reaction to chemotherapy occurring within a 12-month period. Advantageously, a survival model not only assesses the risk of occurrence 42 of the rare event but also provides a temporal dimension, helping clinicians make informed decisions about patient monitoring and treatment adjustment.

It may be observed that the operations by the modules 11, 12, 13, 14, 15, and 16 are not necessarily successive in time, and may overlap, proceed in parallel or alternate, in any appropriate manner.

The device 1 may further comprise a module 17 for providing the at least one trained machine learning model 41. In an embodiment, the trained machine learning model 41 may be provided with at least one performance metric.

In an embodiment, device 1 may be configured for obtaining multiple trained machine learning models 41, wherein each trained machine learning models 41 has been trained using a different augmented dataset 30 or a different model architecture. Each of the trained machine learning models 41 is configured at least to predict a risk of occurrence 42 of the rare event for a patient, specific to the augmented dataset 30 and/or the model architecture used for training. Device 1 may further select one of the obtained trained machine learning models 41based on a comparison of at least one performance metric associated with the trained machine learning models 41 with a predefined performance criterion. Alternatively, the average or a combination of the risk of occurrence 42 outputted by all trained machine learning models 41may be provided as output of device 2. This allows to account for various factors (e.g. such as data variability, and training bias) and select or combine the best model(s) for each individual case.

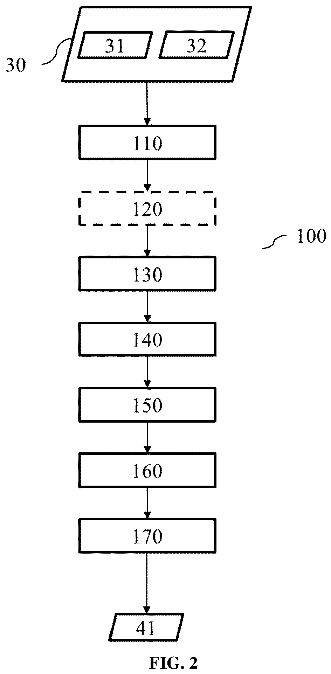

In its automatic actions, the device 1 may for example execute the following computer-implemented method 100 **(****Figure 2**):
- receiving 110 an augmented dataset 30 comprising:
   ∘ a subjects dataset 31 comprising subject data from a plurality of subjects at risk of said at least one rare event and subject data from a plurality of subjects not at risk of said at least one rare event;
   ∘ for each subject of the subjects dataset 31, a set of parameters 32 associated to said at least one rare event, said rare event being associated to at least one initiator event, and/or at least one intermediate event and/or at least one precursor event, said set of parameters 32 comprising: at least one initiator event parameter and/or at least one intermediate event parameter and/or at least one precursor event parameter;
- optionally, a preprocessing step 120, similar to the function of module 12;
- clustering 130 said augmented dataset 30 based on at least one parameter of said set of parameters 32, so as to obtain at least two event clusters, each event cluster of said at least two event clusters being associated to at least one initiator event and/or at least one intermediate event and/or at least one precursor event;
- computing 140 at least one trajectory of weak signal diffusion between said at least two event clusters, and determine, for each computed trajectory, an associated risk score;
- computing 150 at least one distance between said rare event and at least one data of each event cluster of said at least two event clusters;
- training 160 at least one machine learning model using said augmented dataset, said at least one trajectory and the associated risk score, said at least two event clusters and said computed at least one distance, so as to obtain said at least one trained machine learning model 41, said trained machine learning model 41 being configured to receive at least a patient dataset 33 and a patient set of parameters 34 associated to said rare event and to provide said risk of occurrence 42, in a predefined time interval, of at least one rare event for said patient ;
- providing 170 said at least one trained machine learning model 41.

Device 2 may be adapted for predicting for a patient the risk of occurrence 42 of the rare event, in a predefined time interval, using a trained machine learning model 41 obtained using device 1 according to any of the disclosed embodiments.

Device 2 may comprise at least one input (module 21) configured to receive:
- a patient dataset 33 and a patient set of parameters 34 associated to the rare event;
- the trained machine learning model 41.

The patient dataset 33, the patient set of parameters 34, and the trained machine learning model 41 may be stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). Alternatively, the patient dataset 33, the patient set of parameters 34, and the trained machine learning model 41 may be received from a communication network.

The patient dataset 33 may comprise patient data collected from the patient. Patient data refer to information related to a patient's health, collected from various sources, including routine medical check-ups, electronic health records (EHRs), patient self-reported data, wearable medical devices, laboratory tests, imaging studies, genetic analysis, clinical trials, observational studies, and real-time monitoring systems.

Subject data can come in various formats, including numerical, text, categorical, image, audio, video and time-series data.

The patient dataset 33 may comprise the same data as the one collected for the subjects of the augmented dataset 30.

For instance, the patient dataset may comprise a baseline health score (e.g. obtained from a questionnaire, from risk markers, from a tool for assessing the risk of developing breast and ovarian cancer such as CanRisk; demographics like identity, age, genetic risk; diagnoses and treatment information (e.g. hormone replacement therapy for menopause); lab test results, such as blood tests measuring tumor markers or genetic testing for mutations; clinical intervention data, like records of chemotherapy or surgical treatments and associated treatment side effects; lifestyle and environmental data, including information about diet, smoking habits, or exposure to environmental toxins.

The patient set of parameters 34 may comprise at least one of initiator event parameter(s), intermediate event parameter(s) and precursor event parameter(s) such as defined in reference to the set of parameters 32 used for training.

The patient set of parameters 34 may be the same set of parameters as the one used for training.

Device 2 may further comprise optionally a module 22 (similar in function to module 12 of device 1) for preprocessing the received patient dataset 33 and patient set of parameters 34 associated to the rare event.

Device 2 may further comprise a module 23 for predicting the risk of occurrence 42 of the rare event for the patient, by feeding the patient dataset 33 and the patient set of parameters 34 associated to the rare event to the trained machine learning model 41.

The risk of occurrence 42, in a predefined time interval, of at least one rare event for a patient outputted by the trained machine learning model 41 may be at least one of: a probability value, a binary value, a percentage, and a Mean Squared Error.

Device 2 may further comprise a module 24 for providing at least the risk of occurrence 42 of the rare event for the patient.

In an embodiment, the risk of occurrence 42 of the rare event may be compared to a threshold to identify if the patient is at risk of encountering the rare event. In an example, the threshold may be defined as a weighted combination of risk factors, such as genetic predisposition, age, and environmental exposures. In another example, the threshold may defined based on clinical evidence.

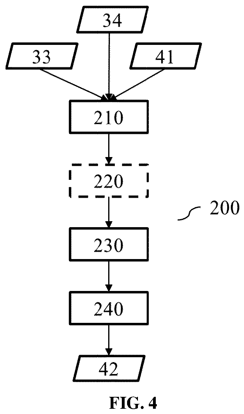

In its automatic actions, the device 1 may for example execute the following computer-implemented method 200 **(****Figure 4****):**
- receiving 210:
   ∘ a patient dataset 33 and a patient set of parameters 34 associated to said rare event;
   ∘ the at least one trained machine learning model 41;
- optionally, a preprocessing step 220, similar to the function of module 12;
- predicting 230 the risk of occurrence 42, in a predefined time interval, of at least one rare event for the patient, by feeding the patient dataset 33 and the patient set of parameters 34 associated to the rare event to the at least one trained machine learning model 41,
- providing 240 at least the risk of occurrence 42, in a predefined time interval, of at least one rare event for the patient.

The devices 1 and/or 2 may interact with a user interface 50, via which information can be entered and retrieved by a user. The user interface 50 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

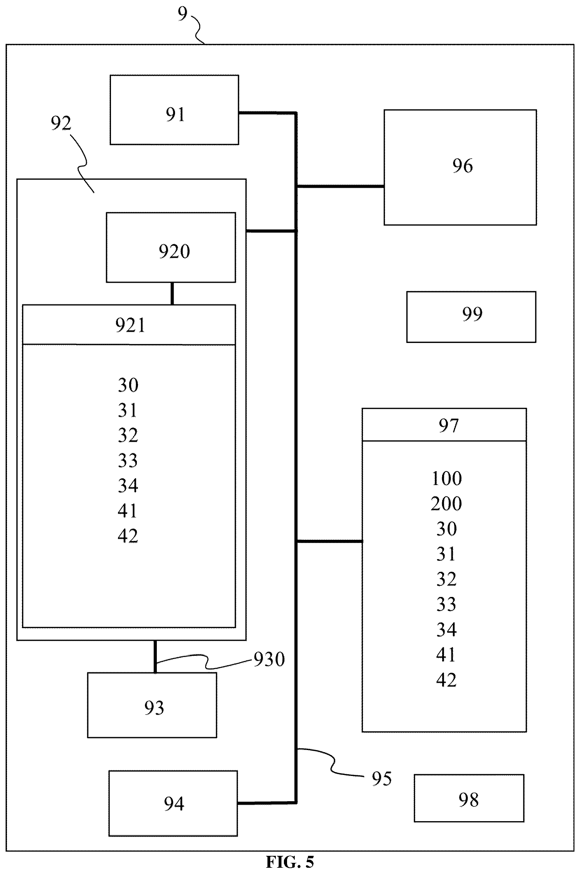

A particular apparatus 9, visible on **Figure 5****,** is embodying the device 1 as well as the device 2 described above. It corresponds for example to a workstation, a laptop, a tablet, a smartphone, or a head-mounted display (HMD).

That apparatus 9 is suited to prediction of the risk of occurrence 42 of a rare event and to related machine learning training. It comprises the following elements, connected to each other by a bus 95 of addresses and data that also transports a clock signal:
- a microprocessor 91 (or CPU);
- a graphics card 92 comprising several Graphical Processing Units (or GPUs) 920 and a Graphical Random Access Memory (GRAM) 921; the GPUs are quite suited to image processing, due to their highly parallel structure;
- a non-volatile memory of ROM type 96;
- a RAM 97;
- one or several I/O (Input/Output) devices 94 such as for example a keyboard, a mouse, a trackball, a webcam; other modes for introduction of commands such as for example vocal recognition are also possible;
- a power source 98; and
- a radiofrequency unit 99.

According to a variant, the power supply 98 is external to the apparatus 9.

The apparatus 9 also comprises a display device 93 of display screen type directly connected to the graphics card 92 to display synthesized images calculated and composed in the graphics card. The use of a dedicated bus to connect the display device 93 to the graphics card 92 offers the advantage of having much greater data transmission bitrates and thus reducing the latency time for the displaying of images composed by the graphics card. According to a variant, a display device is external to apparatus 9 and is connected thereto by a cable or wirelessly for transmitting the display signals. The apparatus 9, for example through the graphics card 92, comprises an interface for transmission or connection adapted to transmit a display signal to an external display means such as for example an LCD or plasma screen or a video-projector. In this respect, the RF unit 99 can be used for wireless transmissions.

It is noted that the word "register" used hereinafter in the description of memories 97 and 921 can designate in each of the memories mentioned, a memory zone of low capacity (some binary data) as well as a memory zone of large capacity (enabling a whole program to be stored or all or part of the data representative of data calculated or to be displayed). Also, the registers represented for the RAM 97 and the GRAM 921 can be arranged and constituted in any manner, and each of them does not necessarily correspond to adjacent memory locations and can be distributed otherwise (which covers notably the situation in which one register includes several smaller registers).

When switched-on, the microprocessor 91 loads and executes the instructions of the program contained in the RAM 97.

As will be understood by a skilled person, the presence of the graphics card 92 is not mandatory, and can be replaced with entire CPU processing and/or simpler visualization implementations.

In variant modes, the apparatus 9 may include only the functionalities of the device 1, and not those of the device 2. In addition, the device 1 and/or the device 2 may be implemented differently than a standalone software, and an apparatus or set of apparatus comprising only parts of the apparatus 9 may be exploited through an API call or via a cloud interface.

### EXAMPLES

### Example 1: a mathematical modeling approach for weak signals.

A weak signal of the incubation of a rare event is the characteristic (signature and/or 1^{st}, ... n^{th} derivative(s) of the mixed probabilistic-deterministic mathematical model of the risk associated with the rare event. This integrated multi-scale model, incorporating AI, implements a combinatorial approach to multi-physical predictive markers of exposure risk to initiator and/or precursor events leading to the dreaded rare event.

A weak signal is characterized at a minimum by its intensity and probability of occurrence. The intensity of the weak signal serves as a predictive marker characteristic of the incubation of the dreaded rare event (i.e. the rare event) (e.g. cancer recurrence, lupus flare-up, therapeutic resistance, industrial event, etc.).

It can also be characterized, for example, by its incidence, the severity level of the anticipated risk, and/or the degree of concomitance. The latter is defined as the number of distinct events that can be observed following the occurrence of the weak signal. For instance, a weak signal triggered by the risk factor *alcoholism* may be associated with multiple concomitant diseases, such as cardiovascular diseases, cancers, or cirrhosis.

A weak signal can be attenuated or amplified by so-called attenuating or amplifying risk factors and may be drowned out or hidden within a cloud of uncertainties.

It can be modeled in several different ways (non-exhaustive list):
- An acoustic, electrical, electromagnetic, or other low-intensity signal, detected, for example, by an ultrasound device or sensors;
- A mathematical object, such as a vector or a risk matrix characteristic of a dreaded event;
- A multivariable mathematical function;
- A characteristic risk heatmap.

The precursor signals fall into four categories:
- Critical signals are at the core of the first peripheral circle of the cluster and are assigned a high level of confidence (narrow confidence interval). They are characterized by a significant history of incident precursor signals from the second circle converging toward them. They are associated with a high alert level, requiring urgent action to contain the impact in response to the imminent realization of the threat or dreaded event (e.g., a major incident or near-accident).
- Strong signals are located in the second peripheral circle of the cluster and are assigned a medium level of uncertainty (wider confidence interval). They are characterized by a non-negligible history of precursor signals from the third circle converging toward them. They are associated with a moderate alert level, requiring rapid action to validate the diagnosis and initiate a corrective and curative action plan in response to the confirmed occurrence of the threat or dreaded event in the near future (e.g., a significant incident or anomaly).
- Weak signals are often isolated and located within or even outside the third peripheral circle of the cluster. These weak signals are generally found outside the previously mentioned clusters but can be associated with a set of clusters representing one or more families of events and/or multiple threat scenarios. They are assigned a low level of confidence (very wide confidence interval). They are not specific to a particular event and require deeper characterization and long-term monitoring to assess their interdependence and correlations with other isolated weak signals. It is also necessary to verify whether they are white signals or statistically insignificant outliers.
- White signals are random signals that must be evaluated using statistical and probabilistic models to confirm that they are not weak signals.

Utilizing the following mathematical functions:
- Distance to the cluster center: A proximity measure to determine the circle.
- History of converging signals: Weighting of converging signals based on their frequency.
- Confidence interval: Calculation based on statistical intervals (e.g., ±1.96σ for 95%).

Based on four classification criteria for signals:
- Critical signals:
   ∘ Located in the first circle.
   ∘ Significant history of converging signals.
   ∘ Narrow confidence interval.
- Strong signals:
   ∘ Located in the second circle.
   ∘ Non-negligible history of converging signals.
   ∘ Wider confidence interval.
- Weak signals:
   ∘ Isolated or outside the third circle.
   ∘ Limited history or weak correlations.
   ∘ Very wide confidence interval.
- White signals:
   o Random noise identified by statistical models.

A weak signal is in motion and changes with each cluster period (Cᵢ). Its propagation function over time allows starting with a broader set of clusters and gradually narrowing this scope to confirm or refute its convergence toward the 3^{rd} circle of the rare cluster.

Its detection relies on its scoring (sigma function) in parallel with comparative matrix analysis against the reference matrix.

To model the kinetics of the weak signal, a probabilistic risk model incorporating a trusted AI (Artificial Intelligence) is integrated, based on the following elements:
- Risk Assessment: Each cluster is quantified based on its frequency, the dispersion of its signals, and their historical relationship.
- Uncertainty Quantification: Confidence intervals are calculated on the data for each signal category (critical, strong, weak, white).
- Trusted AI Implementation: A parallel statistical verification model ensures the validation of critical signals.

To characterize a weak signal, first risk-informed rare event clustering is performed to associate it with its reference cluster(s). Then, its correlation and interdependence with other weak or white signals occurring simultaneously is evaluated, whether they belong to the same cluster or not.

Each weak signal is linked to one or more weak signal clusters, each representing, either dependently or independently, one or more families of rare or significant events.

Weak signals within a cluster may be event-specific or not. A cluster is defined as a mathematical object-numerical, geometric, or topological, open or closed-capable of grouping matrices within the same space. Each matrix models a threat scenario using a mixed deterministic-probabilistic approach. The matrices are binary, oscillating between a null matrix and the identity matrix. Each matrix variable is associated with an occurrence probability and characterized by a confidence interval. At each time step, a new column is created in every matrix, assigning values of 0 or 1 to the risk-predictive variables.

During each period, the prediction of each variable in a cluster matrix column results from the implementation of AI classification algorithms (0/1). These algorithms are pre-trained and enhanced by multi-scale and/or multi-physics risk models associated respectively with the anticipated rare event and adjacent significant events.

Next, associating the characteristic matrix of the weak signal with other matrices is achieved by integrating it into a temporal continuum that includes the historical data of all previous period matrices, with their latest columns completed in a combinatorial and random manner.

The new reference space E(t+1) is then formed, consisting of a portion of the matrices that is deterministically completed by the predictions of the predictive AI algorithms, and another portion that is completed randomly.

Following this first phase, unsupervised AI algorithms are implemented, such as clustering models, to reassess the assignment of the matrix to the initial cluster or reassign it to a new cluster. This implementation runs in parallel with a re-quantification of the associated uncertainties, allowing us to evaluate the confidence level of the clustering process.

In each new cluster, each matrix is modeled using a temporal propagation function, which can be continuous or discontinuous, characterized by an intensity at a given time t, weighted by the frequency, severity, and occurrence probability of the feared rare event, as well as by risk factors that either mitigate or amplify the impact depending on their positive or negative effect.

The first (derivatives) and second (acceleration) partial derivatives of the propagation function are monitored over time, as they model, for each variable, the multidimensional incubation of the feared rare event. This event is characterized by its incidence, a mechanistic function, its occurrence probability, and its severity level. These characteristics of the feared rare event, along with those of other adjacent significant events, are assessed in parallel at each reference period (1 month, 3 months, 6 months, 12 months, 18 months, 24 months, 36 months, 4 years, 5 years), alongside the evaluation of the characteristics of the captured weak signal.

The intensity of a weak signal at a given time t is defined as the square root of the weighted sum of the squares of the differences between the variables at time t, which form the dimensions of the event, and the variables at the reference time t0. The weak signal evaluation is carried out using a mixed deterministic-probabilistic approach, modeling in parallel the deterministic function for calculating intensity and the probabilistic function for calculating the probability of occurrence of the signal.
- No Signal: Intensity strictly below the so-called "White Threshold" and occurrence probability strictly below 0.15%.
- White Signal: A weak, uncharacterized signal with intensity greater than or equal to the "White Threshold" but less than 1.05% of the "White Threshold," and an occurrence probability strictly greater than 0.15% but strictly less than 1.5%. The white signal requires monitoring during the next period and over n periods before confirming or refuting the associated risk: the risk of conflict between two simultaneous scenarios that cancel each other out, for example, a protective scenario and a risk scenario; or a random signal, noise, or an insignificant event that will dissipate on its own after a few reference periods. It must be carefully monitored by modeling the noise and evaluating and quantifying the associated uncertainties.
- Non-characteristic Weak Signal: A signal with moderate intensity greater than or equal to 1.05% of the "White Threshold" but less than 1.10% of the "White Threshold," and an occurrence probability strictly greater than 1.5% but less than 5%. This signal may be associated with multiple scenarios and models. It should be carefully monitored and investigated by evaluating each of the possible scenarios.
- Characteristic Weak Signal: A signal with significant intensity, greater than or equal to 1.10% of the "White Threshold" but less than 1.15% of the "White Threshold," and an occurrence probability strictly greater than but less than 15%. This signal can be associated with a specific scenario and should be investigated thoroughly.
- Nominal Weak Signal: A characterized weak signal with an intensity greater than or equal to 1.15% of the "White Threshold" or with a high probability of occurrence greater than 15%.

The two major challenges posed by a weak signal are its accurate prediction and early detection. To overcome these two obstacles, the solution is based on three modules deployed in parallel and iteratively interacting: its prediction and assessment of its probability of occurrence, analysis of its incidence and adjacent intercorrelations, and quantification of the associated uncertainties. In the current state of the art, a weak signal is detected only when it becomes strong (symptom) or critical (near-accident) in the context of monitoring the exceeding of physical thresholds. A rare event is characterized by unbalanced, low-volume data, generating a significant bias for the learning of AI algorithms.

To solve this problem, a system that automatically generates new, informed, and risk-weighted data is created. It will also deploy, in parallel and iteratively, 6 families of models:
- Multimodal AI algorithms for predicting the probability of occurrence of a weak signal associated with a feared event.
- A multi-scale simulation model for rare events, which will feed into the training database of the aforementioned AI models.
- A mathematical risk model to model the feared risk and measure its impact.
- A model for random phenomena leading to critical events, which will calculate the conditional probabilities of random variables in critical regimes and characterize all uncertainties and correlations that could lead to such events.
- A model for quantifying associated uncertainties to reduce biases and uncertainties, and to increase the confidence in the prediction.
- A model to amplify the detected weak signal to allow its characterization.

The output of this first part is a set of characteristic pre-trained, optimized, and validated AI algorithms for each weak signal category.

Then, the prediction of a characterized early weak signal will rely on the parallel deployment of the 3 best-optimized ML algorithms for each feared event family, and the classification of the weak signal by at least one of the characteristic models.

To detect the characterized weak signal, the risk score is calculated, incidence score, and/or the probability of occurrence over increasingly shorter periods. Then the risk score is derived, which is also calculated over time and derived a second time over the same periods to assess the acceleration.

If the drift and/or acceleration are non-zero, the detection of the characterized weak signal is confirmed.

Example 2: Some potential applications in oncology:
- Early detection of cancer:
   ∘ Identify weak signals that could be early biomarkers for cancer (changes in genetic, metabolic, or cellular profiles).
   ∘ Application to slow-progressing but deadly cancers, such as breast or prostate cancer.
- Monitoring of metastases:
   ∘ Model the spread of metastases from the primary tumor by analyzing incubation pathways.
   ∘ Enable targeted and personalized treatment for at-risk areas.
- Optimization of treatment plans:
   o Adapt treatments based on the anticipated progression of weak signals in surrounding tissues.
   ∘ Reduce unnecessary treatments for low-risk patients and intensify them for critical areas.
- Long-term monitoring:
   ∘ Enable continuous monitoring of patients in remission to detect early signs of relapse.
   ∘ Use multi-scale data (imaging, blood tests, genomic data) to feed into the model.
- Diagnosis of rare clinical situations:
   ∘ Study correlations between rare syndromes and specific types of cancer using unsupervised data.
- Multi-scale modeling:
   o 4D analysis to visualize the evolution of a tumor in an organ, linking biomedical signals at the molecular, cellular, tissue, and organ levels.

Such a solution could become a crucial tool for early diagnosis, risk management, and personalized treatment in oncology. It could also provide a new understanding of the dynamic processes involved in cancer development.

Example 3: Some potential applications for rare diseases:
- Early detection of rare diseases:
   ∘ Identify weak signals in biomedical data that could indicate the onset of a rare disease.
   ∘ Use stochastic models to simulate risk scenarios and detect early patterns that may not be evident in limited data.
- Multi-scale analysis and spatiotemporal modeling:
   ∘ Map the progression of specific symptoms or biomarkers over time and within the body (4D spatiotemporal modeling).
   ∘ Visualize the interaction between different bodily systems to better understand the underlying mechanisms of rare diseases.
- Personalized diagnostics:
   ∘ Integrate patient-specific data (genomics, medical imaging, medical history) for customized analysis.
   ∘ Provide personalized predictions to assess risks and adapt treatment plans.
- Identification of critical biomarkers:
   ∘ Detect weak signals from genetic, metabolic, or environmental data.
   ∘ Identify specific risk factors by clustering comorbidities and optimizing constraints.
- Longitudinal tracking and continuous monitoring:
   ∘ Track patients with rare diseases or predispositions in real time.
   ∘ Detect any subtle or progressive changes through predictive monitoring based on risk scores.
- Optimization of clinical trials:
   ∘ Help design clinical trials for rare diseases by identifying eligible patients and assessing treatment efficacy.
   ∘ Simulate disease trajectories to predict treatment response.
- Improvement of treatment protocols:
   ∘ Based on the propagation of weak signals, adjust treatments to limit the risk of disease progression or complications.
   ∘ Reduce invasive or unnecessary interventions through precise predictive models.
- Prediction of rare complications:
   ∘ Identify patients at risk of rare complications associated with certain diseases or procedures.
   ∘ Integrate intermediate trajectories to alert doctors to the possibility of a rare event.
- Management of public health data:
   ∘ Enable the aggregated analysis of data on rare diseases to better understand their epidemiology.
   ∘ Identify geographical or environmental clusters of rare diseases.
- Decision support for physicians:
   ∘ Provide synthesized reports on risks, trajectories, and critical factors to assist clinicians in making informed decisions.
   ∘ Integrate explanatory models to enhance the understanding of outcomes.

Example 4: Mixed deterministic-probabilistic modeling of a weak signal incubation for a rare event

A weak incubation signal of a rare event is the characteristic (signature and/or 1^{st}... n^{th} derivatives) of a mixed probabilistic-deterministic mathematical model of the risk associated with the rare event. This integrated multiscale model, embedding AI, implements a combinatorial approach to multiphysical markers predictive of risk of exposure to initiating events and/or precursors to the feared rare event. ${σ}_{m} \left({α}_{ω}, {β}_{ω}, x, t\right) = {σ}_{0} + \frac{1}{n} {\sum }_{k = 0}^{n} {δ}_{km} \left(x, t\right) {α}_{ω}^{k} {β}_{ω}^{n - k} {ϕ}_{km} {Ω}_{k}^{ζ}$
σₘ : Time signature of state ω, characterizing the incubation propagation of the rare event during period m;
α_{ω} : a state ω characterizes the patient's condition at a given time t. For example, the patient is undergoing chemotherapy and therefore exposed to specific risks, or is in remission, or is in good health;
β_{ω} : amplification function of the state ω;
x: multi-physical and/or multi-dimensional reference variables, associated with state-of-the-art risk markers;
t: time function for predictive and preventive remote monitoring;
σ₀ : signature of the initial state, validated by a domain expert or calculated by a reference model from qualified initial data;
n: number of markers predictive of risk of exposure to initiating and/or precursor events;
δₖₘ : simplified impact function (1 or 0 or -1) or complex system characteristic;
${α}_{ω}^{k}$: attenuation;
ϕₖₘ : probability of occurrence of the rare event (dreaded event);
${Ω}_{k}^{ζ}$: exposure to the dreaded risk, defined as the estimated Severity level of the kth risk factor and its impact on the occurrence of the dreaded event; ζ being the degree of severity (power > = 1).
Example of impact matrix of n rows and m columns: $\left[\begin{matrix}{δ}_{11} & ⋯ & {δ}_{1 m} \\ ⋮ & ⋱ & ⋮ \\ {δ}_{n 1} & ⋯ & {δ}_{nm}\end{matrix}\right]$ wherein, the n^{th} row represents the impact line for the n^{th} risk factor:
1. Either the risk factor is not characterized, so the value is zero
2. Or the risk factor is characterized, and the value is equal to 1
3. Or the risk factor is corrected by a protective or corrective factor that mitigates risk exposure, so the value is equal to -1.
and wherein the m^{th} column represents the impact column for each new period m.

## Claims

1. A device (1) for obtaining at least one trained machine learning model (41) configured at least to predict a risk of occurrence (42), in a predefined time interval, of at least one rare event for a patient, said device comprising:
- at least one input configured to receive (11) an augmented dataset (30) comprising:
∘ a subjects dataset (31) comprising subject data from a plurality of subjects at risk of said at least one rare event and subject data from a plurality of subjects not at risk of said at least one rare event;
∘ for each subject of the subjects dataset (31), a set of parameters (32) associated to said at least one rare event, said rare event being associated to at least one initiator event, and/or at least one intermediate event and/or at least one precursor event, said set of parameters (32) comprising: at least one initiator event parameter and/or at least one intermediate event parameter and/or at least one precursor event parameter;
- at least one processor configured to:
∘ cluster (13) said augmented dataset (30) based on at least one parameter of said set of parameters (32), so as to obtain at least two event clusters, each event cluster of said at least two event clusters being associated to at least one initiator event and/or at least one intermediate event and/or at least one precursor event;
∘ compute (14) at least one trajectory of weak signal diffusion between said at least two event clusters, and determine, for each computed trajectory, an associated risk score;
∘ compute (15) at least one distance between said rare event and at least one data of each event cluster of said at least two event clusters;
∘ train (16) at least one machine learning model using said augmented dataset (30), said at least one trajectory and the associated risk score, said at least two event clusters and said computed at least one distance, so as to obtain said at least one trained machine learning model (41), said trained machine learning model (41) being configured to receive at least a patient dataset (33) and a patient set of parameters (34) associated to said rare event and to provide said risk of occurrence (42), in a predefined time interval, of at least one rare event for said patient;
- at least one output configured to provide (17) said at least one trained machine learning model (41).

2. The device according to claim 1, wherein each of said at least one initiator event parameter and/or said at least one intermediate event parameter and/or said at least one precursor event parameter is at least one of: a frequency, a duration, a magnitude, and a time information related to : an initiator event associated with said one initiator event parameter, or an intermediate event associated with said one intermediate event parameter, or a precursor event associated with said one precursor event parameter.

3. The device according to any one of claims 1 to 2, wherein said subject data of the subjects dataset (31) comprises for each subject at least one of an age, a genetic risk related to said at least one rare event, a treatment information, and a baseline health score.

4. The device according to any one of claims 1 to 3, wherein clustering (13) said augmented dataset (30) comprises using a k-means approach and/or a DBSCAN approach and/or decision trees and/or a random forest.

5. The device according to any one of claims 1 to 4, wherein computing (14) at least one trajectory of weak signal diffusion comprises using at least one of: a random walk model (e.g. Markov Chain), a graph-based diffusion model, and a differential equation based model.

6. The device according to any one of claims 1 to 5, wherein computing (15) at least one distance comprises calculating an average distance or a median distance or a midrange distance of said rare event to each data of each event cluster of said at least two event clusters.

7. The device according to any one of claims 1 to 6, wherein said at least one machine learning model is at least one of: a Random Forest Classifier, a Gradient Boosting Classifier, a Logistic Regression model, and a SVM classifier.

8. The device according to any one of claims 1 to 7, wherein said at least one machine learning model is a survival model and said risk of occurrence (42), in a predefined time interval, of at least one rare event for a patient is associated with a survival time.

9. The device according to any one of claims 1 to 8, wherein said augmented dataset (30) is previously obtained by stochastic simulation.

10. The device according to claim 9, wherein said stochastic simulation comprises:
- adding at least one subject stochastic component to at least one subject data comprised in said subjects dataset (31); and/or
- adding at least one event stochastic component to at least one of said set of parameters (32) for at least one subject of the subjects dataset (31).

11. The device according to claim 10, wherein adding at least one subject stochastic component comprises using a random number generator or a Monte Carlo simulation.

12. The device according to any one of claims 10 to 11, wherein adding an event stochastic component to said at least one of said sets of parameters (32) comprises using a random event generator.

13. A device (2) for predicting a risk of occurrence (42), in a predefined time interval, of at least one rare event for a patient using at least one trained machine learning model (41) obtained using said device (1) for obtaining at least one trained machine learning model according to any one of claims 1 to 12, said device (2) comprising:
- at least one input configured to receive (21):
∘ a patient dataset (33) and a patient set of parameters (34) associated to said rare event;
∘ said at least one trained machine learning model (41);
- at least one processor configured to predict (23) said risk of occurrence (42), in a predefined time interval, of at least one rare event for said patient, by feeding said patient dataset (33) and said patient set of parameters (34) associated to said rare event to said at least one trained machine learning model (41),
- at least one output configured to provide (24) at least said risk of occurrence (42), in a predefined time interval, of at least one rare event for said patient.

14. The device (2) according to claim 13, wherein said risk of occurrence (42), in a predefined time interval, of at least one rare event for a patient is at least one of: a probability value, a binary value, a percentage, and a Mean Squared Error.

15. The device (2) according to any one of claims 13 to 14, wherein said risk of occurrence (42), in a predefined time interval, of at least one rare event for a patient is compared to a threshold to identify if said patient is at risk of encountering said at least one rare event.
